# EUROPÄISCHE PATENTSCHRIFT

(11) **EP 1 066 820 B1**
(45) Veröffentlichungstag und Bekanntmachung des Hinweises auf die Patenterteilung: **09.03.2005**
(21) Anmeldenummer: 00111127.7
(22) Anmeldetag: 24.05.2000
(51) Int. Cl.: A61K 7/06, A61K 7/48

(54) **Pulverförmige Wirkstoffzusammensetzung, Verfahren zu deren Herstellung und Verwendung derselben**
Power composition, method or preparation and use
Composition en poudre, procédé de préparation and utilisation

(30) Priorität: 07.07.1999 DE 19931271
(43) Veröffentlichungstag der Anmeldung: 10.01.2001
(73) Patentinhaber: KPSS-Kao Professional Salon Services GmbH, 64297 Darmstadt (DE)
(72) Erfinder: Grit, Mustafa, Dr., 64579 Gernsheim (DE)

(56) Entgegenhaltungen:
- WO-A-97/38673
- FR-A- 2 326 177
- US-A- 5 520 908
- DATABASE WPI Derwent Publications Ltd., London, GB; AN 1994-354596 XP002256107 NN: "Powder composition for use in hair care cosmetics" & JP 06 279233 A (HOYU KK), 4. Oktober 1994 (1994-10-04)

## Beschreibung

Die vorliegende Erfindung betrifft eine Wirkstoffzusammensetzung, die aufgrund ihrer haut- und haarpflegenden Eigenschaften insbesondere zur Anwendung in kosmetischen Mitteln geeignet ist, und ein Verfahren zu deren Herstellung.

Es ist seit langem bekannt, in Haut- und insbesondere Haarpflegemitteln, vorzugsweise in Form wäßriger Emulsionen, quaternäre Ammoniumverbindungen, die mindestens eine langkettige Alkylgruppe enthalten, im Gemisch mit öl- und fettartigen pflegenden Substanzen wie Fettalkoholen einzusetzen.

Diese Emulsionen wirken auf Haut und Haar strukturverbessernd und sind besonders zur Behandlung und Regenerierung von strukturgeschädigtem Haar geeignet.

Sie konditionieren das Haar und verbessern dessen Elastizität, die Naß- und Trokkenkämmbarkeit und verleihen ihm erhöhten Glanz und Volumen.

Es wurde jedoch festgestellt, daß diese Emulsionen nicht in allen Zubereitungsformen über längere Zeit stabil bleiben und es auch, je nach Anwendungsform und -art und erstrebter Wirkung, nicht immer möglich ist, die geeignete Dosierung zu wählen, was unnötige Kosten bei einer Überdosierung verursacht, andererseits aber bei erwünschten Höherdosierungen Einarbeitungsprobleme hervorrufen kann. Die Erfindung hat sich die Aufgabe gestellt, diese Nachteile zu vermeiden.

JP 06 279 233 A offenbart pulverformige Kosmetikzusammensetzungen die zur Haarkonditionierung geeignet sind. In der FR 2 326 177 A wird eine pulverformige Haarpflegezusammensetzung beschrieben, die Polymere enthält. Keinem der beiden Dokumente ist eine Zusammensetzung zu entnehmen, die Ester oder Amido Quat enthält.

Die Lösung dieser Aufgabe besteht darin, eine wasserlösliche oder wasserdispergierbare pulverförmige Wirkstoffzusammensetzung, deren Partikel zu mindestens 90 Gew.-% einen mittleren Teilchendurchmesser zwischen 10 und 500 µm aufweisen, zu verwenden, die
(i) 5 bis 90 Gew.-% einer Kombination aus
   a) mindestens einer haarkonditionierenden quaternären Ammoniumverbindug ausgewählt aus einer der beiden formel I oder II 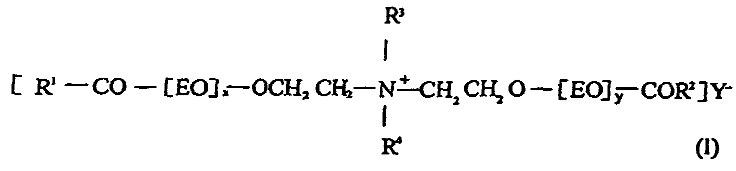 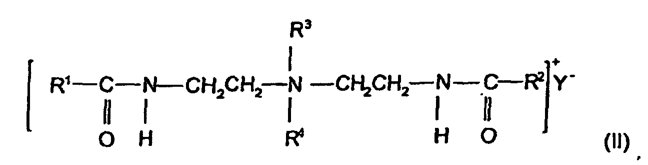 in der R¹ und R² jeweils für eine gegebenenfalls hydroxysubstituierte C₈-C₂₂-Alkyl oder Alkenylgruppe, R³ und R⁴ für eine C₁-C₃-Alkylgruppe oder eine Gruppe -CH₂-CH₂-O-[EO]ₓ-H, sowie x und y für 0 bis 5, und Y für ein Anion stehen, vorzugsweise in einer Menge von 1 bis 35 Gew.-%, und
   b) mindestens einem C₈-C₂₄-Fettalkohol und/oder eine fett- oder ölartige Substanz, vorzugsweise in einer Menge von 1 bis 35 Gew.-%; und
(ii) 10 bis 95 Gew.% mindestens eines wasserlöslichen oder wasserdispergierbaren Bindemittels enthält.

Aus der älteren DE-A 199 11 106 sind bereits pulverförmige Wirkstoffzusammensetzungen bekannt, die mindestens ein Ceramid enthalten.
Derartige Zusammensetzungen sind von der vorliegenden Erfindung nicht umfaßt.

Das erfindungsgemäße pulverförmige Produkt als auch die in diesem enthaltene Wirkstoffkombination können selbstverständlich weitere an sich bekannte Zusatzund Wirkstoffe für kosmetische Mittel enthalten.

Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.
Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.
Diese Verbindungen sind an sich bekannt und beispielsweise unter den Handelsnamen "Schercoquat®", "Dehyquart® F30" und "Tetranyl®" im Handel.
Der Einsatz dieser Verbindungen, sogenannter "Esterquats", in Haarpflegemitteln ist ebenfalls bereits bekannt und beispielsweise in der WO-A 93/10748, der WO-A 92/06899 und der WO-A 94/16677 beschrieben.

Bevorzugte Reste R¹ und R² sind hierbei C₁₂-C₁₈-Alkyl- und Alkenylreste, der Rest R³ eine Methyl- oder Ethylgruppe, und der Rest R⁴ eine Gruppe -CH₂-CH₂-O-[EO]ₓ-H, worin x 0 bis 3 ist; Y⁻ ist vorzugsweise ein Methylsulfat-, Ethylsulfat-, Chlorid- oder Phosphatanion.
Entsprechende Handelsprodukte sind unter der Bezeichnung "INCROQUAT® HO" oder "OCS" bekannt
Die Einsatzkonzentration der quaternären Ammoniumverbindungen in Kombination (i) liegt vorzugsweise bei 2,5 bis 25 Gew.-%.

Geeignete fett- und ölartige Substanzen, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.
Synthetische Öle und Wachse sind beispielsweise Silikonöle, Polyethylengykole, etc. Weitere geeignete hydrophobe Komponenten sind insbesondere Fettalkohole mit 8 bis 24 Kohlenstoffatomen im Molekül wie Lauryl-, Cocoyl-, Myristyl-, Cetyl-, Plamityl-, Oleyl-, Behenyl- und Stearylalkohol, Wachsalkohole und Fettsäureester wie Isopropylmyristat, -palmitat, -stearat und -isostearat, Oleyloleat, tsocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in der erfindungsgemäß verwendeten Zusammensetzung (i) vorzugsweise in einer Gesamtmenge von etwa 1 bis etwa 35, insbesondere etwa 2 bis 25, vor allem etwa 2,5 bis 20 Gew.-% enthalten.
Gemäß einer bevorzugten Ausführungsform der Erfindung enthält die erfindungsgemäße pulverförmige Zusammensetzung noch Harnstoff und/oder ein wasserlösliches Erdalkalisalz, vorzugsweise in einer Menge von etwa 1 bis 35, insbesondere etwa 5 bis 25 Gew.-% des Pulvers.
Geeignete Erdalkalisalze sind insbesondere Calciumchlorid und Magnesiumchlorid sowie Magnesiumsulfat. Diese Stoffe sind bekannte Wirkstoffe für Haut- und Haarpflegemittel.

Die erfindungsgemäßen Zusammensetzungen können auch Tenside, vorzugsweise nichtionische Emulgatoren enthalten, beispielsweise in einer Menge von etwa 1 bis etwa 30 Gew.-% des Pulvers.
Als nichtionische Tenside sind C₈-C₂₀-Alkylglucoside bevorzugt, vor allem solche mit einem Kondensationsgrad (z) von etwa 1,1 bis etwa 5, vorzugsweise in einer Menge von etwa 1 bis 15 Gew.-% der Zusammensetzung.
Weitere bevorzugte nichtionische Tenside sind C₁₂-C₁₈-Fettalkohol-Ethylenoxid-Kondensate wie Laureth-6, Laureth-12, Coceth-8, Myristeth-6, -9, -12, -15, -20, Ceteth-5, Steareth-8, -10, -20, -22, etc.
Es können auch weitere nichtionische Tenside, allein oder im Gemisch mit Alkylpolyglucosiden und Fettalkoholethoxylaten, eingesetzt werden, beispielsweise Polyolfettsäureester, Sorbitanester und/oder Aminoxide.

Die erfindungsgemäß verwendete Kombination kann auch Sterole enthalten.
Diese können tierischen Ursprungs sein (Zoosterine), z.B. Cholesterol und Lanosterol.
Besonders bevorzugt sind jedoch Phytosterole, d.h., Sterole pflanzlichen Ursprungs. Als solche seinen beispielhaft Ergosterol, Sitosterol, Stigmasterol, Fucosterol, Brassicasterol, Fungisterol, Campesterol, Zymosterol, Ascosterol, Cerevisterol, Episterol, Faecosterol, Spinasterol oder auch Gemische von Phytosterolen, z.B. Sojasterol, genannt.
Ein im Rahmen der Erfindung besonders bevorzugtes Phytosterol ist "Avocadin", d.h., die in der unverseifbaren Fraktion des Avocadoöls vorliegenden Phytosterole. Generell ist festzustellen, daß alle in pflanzlichen Fetten, Ölen und Wachsen vorhandenen Phytosterine bzw. Phytosteringemische zum erfindungsgemäßen Einsatz geeignet sind.

Deren Anteil in der Wirkstoffkombination (i) liegt, wenn vorhanden, bevorzugt bei etwa 1 bis 35, insbesondere bei etwa 2,5 bis 25, insbesondere etwa 5 bis 15 Gew.-%

Geeignete Bindemittel zur Herstellung des erfindungsgemäßen Wirkstoffagglomerats und zur Durchführung des erfindungsgemäßen Verfahrens sind im Prinzip alle wasserlöslichen bzw. wasserdispergierbaren, für diesen Zweck bekannten natürlichen und synthetischen Polymeren.

Als solche sind insbesondere die verschiedenen Cellulose-Derivate, beispielsweise Alkylcellulosen wie Methylcellulose und Ethylcellulose, Hydroxyalkylcellulosen wie Hydroxyethylcellulose, Hydroxypropylcellulose und Methylhydroxypropylcellulose sowie auch Carboxymethylcellulose genannt; weiterhin Stärke und Stärkederivate;Gelatine, natürliche Gummen wie Guar-Gum oder Xanthan-Gum oder auch Alkalialginate und Alginsäureester, beispielsweise Propylenglykolalginat, sowie Polyethylenglykole mit einem Molgewicht ab 10.000.
Geeignete synthetische Polymere sind beispielsweise Polyvinylpyrrolidon, Polyacrylamid, Alkalisalze der Polyacrylsäure, etc.
Besonders geeignet als Bindemittel sind Dextrine und Dextrinderivate wie weißes und gelbes Dextrin und Cyclodextrin sowie insbesondere Maltodextrin.

Die obengenannten Bindemittel können gleichzeitig eine verdickende Wirkung aufweisen.
Weitere geeignete Bindemittel sind beispielsweise Acrylestercopolymerisate mit Acrylsäure und/oder Dialkylaminoalkyl(meth)acrylaten, beispielsweise Diethyl- und Dimethylaminoethylmethacrylat, die gegebenenfalls auch quaternisiert sein können, neutralisierte Copolymerisate aus Methacrylsäure und Methylmethacrylat sowie weitere bekannte wasserlösliche und wasserdispergierbare Copolymere.

Die Gesamtmenge des im Pulver vorhandenen Bindemittels liegt bei 10 bis 95 Gewichtsteilen, insbesondere 20 bis 70 Gewichtsteilen, vorzugsweise 25 bis 50 Gewichtsteilen im Verhältnis zu 5 bis 90 Gewichtsteilen, insbesondere 30 bis 80 Gewichtsteilen der Wirkstoffkombination im sprühgetrockneten Wirkstoffpulver.

Die Herstellung der erfindungsgemäßen Pulver erfolgt vorzugsweise durch homogenes Vermischen wäßriger Emulsionen der Wirkstoffkombination und des Bindemittels, und anschließendes Sprühtrocknen der homogenen Mischung bei erhöhter Temperatur, insbesondere zwischen etwa 60°C (Ausgangstemperatur) und etwa 200 °C (Eingangstemperatur).
Geeignete Sprühtrockenverfahren und Vorrichtungen zu deren Durchführung sind aus dem Stand der Technik hinreichend bekannt.

Die so erhaltenen wasserlöslichen bzw. wasserdispergierbaren Wirkstoffpulver enthalten weniger als 10 Gew.-% Teilchen, deren mittlerer Teilchendurchmesser unter 10 oder über 500 µm liegt.

Vorzugsweise liegen 90 Gew.-% der Teilchen im Bereich zwischen etwa 25 und 400 µm, insbesondere etwa 50 bis 400 µm, so daß es sich um ein staubfreies Pulver handelt.
Die Teilchengröße ist abhängig von der Art der Sprühtrocknung und der dabei eingesetzten Sprühdüse.
Die Bestimmung des durchschnittlichen Teilchendurchmessers kann auf übliche Weise erfolgen, z.B. durch Siebanalyse oder im Laserverfahren.
Eine 10%-ige wäßrige Dispersion oder Lösung dieser Teilchen in Wasser weist vorzugsweise einen pH-Wert im sauren bis neutralen Bereich, beispielsweise zwischen etwa 3 und 7, auf.

Die erfindungsgemäße pulverförmige Wirkstoffmischung, die auch noch weitere in kosmetischen Mitteln übliche Wirk- und Zusatzstoffe enthalten kann, wird vorzugsweise in Haarpflegemitteln eingesetzt.

Für den Einsatz der beschriebenen Kombination sind insbesondere Haarbehandlungsmittel wie Dauerwellmittel einschließlich Vor- und Nachbehandlungsmitteln, Haarfärbemittel auf Basis direktziehender und Oxidations-Farbstoffe, peroxidhaltige Fixiermittel für Dauerwellen bzw. Entwickler für Oxidationshaarfarben, Haamachbehandlungsmittel, die entweder im Haar verbleiben oder nach der Behandlung ausgespült werden, beispielsweise Haarspülungen, die auch direktziehende Farbstoffe enthalten können, Haarkuren, Haarkonditioner, die zusätzlich weitere bekannte konditionierende Wirkstoffe wie anionische, kationische, amphotere und/oder nichtionische Polymere, etc. enthalten können, Haarwässer und Haarfestiger geeignet.

Die Zusammensetzung der Haarpflegemittel, in denen die erfindungsgemäße Kombination verwendet wird, ist grundsätzlich bekannt.
Es wird hierzu, zur Vermeidung von Wiederholungen, auf die umfassende Monographie von K. Schrader, Grundlagen und Rezepturen der Kosmetika, 2. Aufl. (1989, Hüthig Buchverlag), verwiesen.

Dort sind auch Hautkosmetika, deren Zusammensetzung und Herstellung auf S. 387 bis 620 detailliert beschrieben.
Haarkosmetische Präparate, deren Zusammensetzung und Herstellung sind auf S. 677 bis 848 geoffenbart.
Rasierhilfsmittel sind schließlich auf S. 867 bis 880 beschrieben.

Die bei Schrader beispielhaft beschriebenen sowie generell die aus dem Stand der Technik bekannten Produkte sind grundsätzlich zur Aufnahme der erfindungsgemäß verwendeten Wirkstoffkombination geeignet.

Die Anwendung erfolgt in der Weise, daß in eine wäßrige Zusammensetzung bekannter Art vor der Anwendung die gewünschte Menge an Wirkstoffpulver eingebracht, durch Schütteln oder Rühren eine intensive Mischung hergestellt und diese dann auf dem Substrat, z.B. Haut oder Haar, angewendet wird.
Alternativ dazu können die Wirkstoffpulver auch in Wasser gelöst oder dispergiert und direkt angewendet werden, beispielsweise auch als Vor- oder Nachbehandlungsmittel.

Die Menge der Wirkstoffkombination in den Endprodukten hängt von deren Zusammensetzung und dem angestrebten Effekt ab.
Prinzipiell kommt ein Rahmen von etwa 0,01 bis etwa 25, vorzugsweise etwa 0,1 bis 20, insbesondere 0,5 bis 15 Gew.-%, bezogen auf das Endprodukt und den Wirkstoffgehalt der erfindungsgemäßen Zusammensetzung, in Betracht.
Der pH-Wert der Endprodukte liegt vorzugsweise im sauren bis leicht alkalischen Bereich bei etwa 2,5 bis etwa 8, vorzugsweise zwischen 3 und 7.

Die folgenden Beispiele dienen der Illustration der Erfindung.

Es wurde eine erfindungsgemäße Wirkstoffkombination A-1 der folgenden Zusammensetzung hergestellt:

### Zusammensetzung A-1:

70 g einer wäßrigen Emulsion aus einer Verbindung der Formel I

| | |
|---|---|
| (R¹ = R²= Oleyl; R³ = CH₃; R⁴ = CH₂-CH₂-OH; Y⁻ = CH₃SO₄⁻) | 1,0 |
| Steartrimoniumchlorid | 6,0 |
| Cetylstearylalkohol | 3,0 |
| C₁₂-C₁₄-Alkylpolyglucosid (z ∼ 1,5) | 1,5 |
| Laureth-12 | 0,5 |
| Wasser | 15 |

wurden mit 30 g Maltodextrin (Maltrin®M040) in 50 g Wasser und einer Zusammensetzung von 8 g Harnstoff und 8 g CaCl₂ in 16 g H₂O homogen vermischt und dann die erhaltene Mischung in einem üblichen Sprühtrockner bei einer Eingangstemperatur von etwa 170 bis 200°C und einer Ausgangstemperatur von etwa 70 bis 90°C sprühgetrocknet.
Es wurde ein wasserdispergierbares Pulver mit einem Teilchengrößenbereich von > 90 Gew.-% der Partikel zwischen 50 und 400 µm erhalten.
Anstelle des Maltodextrins kann mit gleichem Resultat als Bindemittel auch Hydroxyethylcellulose eingesetzt werden.

### Beispiel 1

| **Pulver** | |
|---|---|
| "Zusammensetzung A-3" | 55 (g) |
| Mikrokristalline Cellulose (Avicel® PH 100) | 11 |
| Citronensäure | 17 |
| Natriumbicarbonat | 17 |

Dieses Pulver dispergiert unter CO₂-Entwicklung in 200 g Wasser und kann als Haarbehandlungsmittel mit einer vorzüglichen Konditionierwirkung eingesetzt werden.

### Beispiel 2

| **Haar- und Kopfhautlotion** | |
|---|---|
| Acrylsäure/C₁₀-C₃₀-Alkylacrylat-Copolymer | 0,20 |
| Natriumhydroxid | 0,02 |
| Panthenol | 0,05 |
| Isostearoyllactylat | 0,05 |
| 1,2-Propandiol | 5,00 |
| Ethanol | 20,00 |
| PEG-160-hydriertes Ricinusöl | 0,30 |
| Parfum, Citronensäure | q.s. |
| Wasser | ad 100,0 |

20 ml dieses Haarwassers wurden vor der Anwendung 5 g der "Zusammensetzung A-3" (hergestellt mit Hydroxyethylcellulose als Bindemittel) intensiv zugemischt. Nach dem Aufbringen dieser Mischung auf Haar und Kopfhaut zeigte das Haar Glanz, Volumen und Elastizität.

### Beispiel 3

Eine Emulsion aus

| | |
|---|---|
| Coceth-10 | 2,0 |
| Avocadin | 2,0 |
| Steartrimoniumchlorid | 6,0 |
| Verbindung der Formel I | 5,0 |
| (R¹ = R² = Stearyl; R³= CH₃; | |
| R⁴= -CH₂-CH₂-OH; Y⁻ = CH₃SO₄⁻) | |
| Maltodextrin | 5,0 |
| Wasser | ad 100 (Gew.-%) |

wurden in der in Beispiel A-1 beschriebenen Weise sprühgetrocknet

10g des erhaltenen Pulvers wurden in 50 g Wasser eingerührt.

Es wurde eine Dispersion mit ausgezeichneten Haarkonditioniereigenschaften erhatten, die als Nachbehandlungsrnirtel eingesetzt wurde.

### Beispiel 4

| | |
|---|---|
| Ceteth-20 | 2,0 |
| Behenyltrimoniumchlorid | 6,0 |
| Mandelöl | 2,5 |
| Verbindung der Formel II (R¹ = R² = Oleyl; R³ = CH₃; R⁴= -CH₂-CH₂-OH; Y⁻ = CH₃SO₄⁻) | 5,0 |
| Hydroxyethylcellulose | 25,0 |
| Wasser | ad 100 (Gew.-%) |

wurde in der in Beispiel A-1 beschriebenen Weise sprühgetrocknet.

Dieses Pulver wurde im Gewichtsverhältnis 1 zu 5 in Wasser dispergiert.

Es wurde ein Produkt mit ausgezeichneten haarkonditionierenden Eigenschaften erhalten.

## Patentansprüche

1. Pulverförmige Wirkstoffzusammensetzung, enthaltend
**(i)** 5 bis 90 Gew.-% einer Kombination aus
a) mindestens einer haarkonditionierenden quaternären Ammoniumverbindung, ausgewählt aus einer der beiden Formeln I oder II 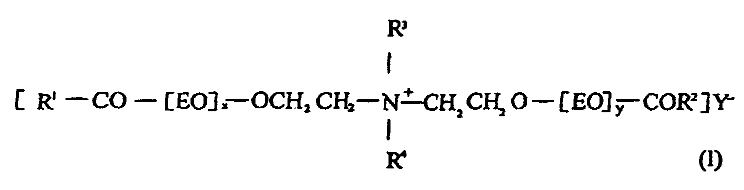 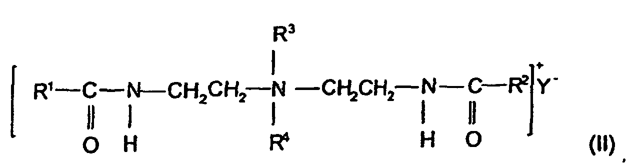 in der R¹ und R² jeweils für eine gegebenenfalls hydroxysubstituierte C₈-C₂₂-Alkyl oder Alkenylgruppe, R³ und R⁴ für eine C₁-C₃-Alkylgruppe oder eine Gruppe -CH₂-CH₂-O-[EO]ₓ-H, sowie x and y für 0 bis 5, und für ein Anion stehen, und
b) mindestens einem C₈-C₂₄-Fettalkohol und/oder einer fett- oder ölartigen Substanz; und
**(ii)** 10 bis 95 Gew.-% mindestens eines wasserlöslichen oder wasserdispergierbaren Bindemittels, wobei mindestens 90% der Partikel einen mittleren Teilchendurchmesser von etwa 10 bis 500 µm aufweisen.

2. Zusammensetzung nach Anspruch 1, enthaltend eine Kombination (i) aus
a) 1 bis 35 Gew.-% mindestens einer quaternären Ammoniumverbindung,
b) 1 bis 35 Gew.-% mindestens eines C₈-C₂₄-Fettalkohols und/oder Fetts oder Öls; und
c) 1 bis 30 Gew.-% mindestens eines nichtionischen Tensids,
jeweils berechnet auf die Zusammensetzung der Kombination.

3. Zusammensetzung nach Anspruch 1 oder 2, **dadurch gekennzeichnet, daß** sie als wasserlösliches Bindemittel 15 bis 70 Gew.-% Maltodextrin enthält.

4. Zusammensetzung nach einem oder mehreren der Ansprüche 1 bis 3, **dadurch gekennzeichnet, daß** sie zusätzlich etwa 1 bis 35 Gew.-% Harnstoff enthält.

5. Zusammensetzung nach einem oder mehreren der Ansprüche 1 bis 4, **dadurch gekennzeichnet, daß** sie zusätzlich 1 bis 35 Gew.-% mindestens eines Erdalkalisalzes enthält.

6. Verfahren zur Herstellung einer pulverförmigen Zusammensetzung nach einem oder mehreren der Ansprüche 1 bis 5, **dadurch gekennzeichnet, daß** eine wäßrige Zusammensetzung, die 5 bis 30 Gewichtsteile einer als wäßrige Emulsion vorliegende Wirkstoffkombination (i) nach den Ansprüchen 1 bis 5 enthält, mit einer wäßrigen Zusammensetzung, die 10 bis 95 Gewichtsteile mindestens eines wasserlöslichen oder wasserdispergierbaren Bindemittels enthält, vermischt, und diese Mischung bei erhöhter Temperatur homogenisiert und anschließend sprühgetrocknet wird.

7. Verwendung einer pulverförmigen Zusammensetzung nach einem oder mehreren der Ansprüche 1 bis 5 in Haarpflege- und Haarbehandlungsmitteln.

## Claims

1. Pulverulent agent composition, comprising
(i) 5 % to 90 % by weight of a combination of
a) at least one hair-conditioning quaternary ammonium compound selected from the compounds represented with the general formula I or II 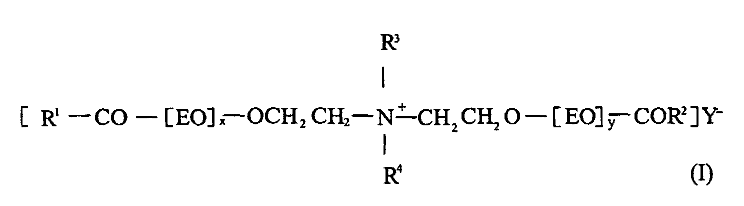 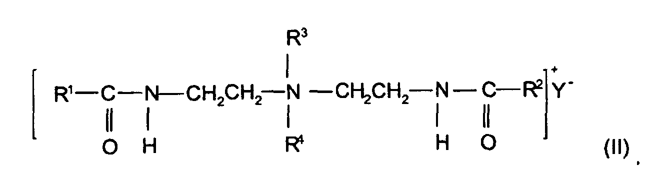 wherein R¹ and R² each stand for an optionally hydroxy-substituted C₈-C₂₂-alkyl or alkenyl group, R³ and R⁴ stand for a C₁-C₃-alkyl group or a group -CH₂-CH₂-O-[EO]ₓ-H, x and y stand for 0 to 5, and Y⁻ stands for an anion, and
b) at least one C₈-C₂₄-fatty alcohol and/or a fatty or oily substance; and
(ii) 10 % to 95 % by weight of at least one water-soluble or water-dispersible binding agent, whereby at least 90 % of the particles have an average particle diameter of about 10 to 500 µm.

2. Composition according to claim 1, comprising a combination (i) of
a) 1 % to 35 % by weight of at least one quaternary ammonium compound
b) 1 % to 35 % by weight of at least one C₈-C₂₄-fatty alcohol and/or fat or oil; and
c) 1 % to 30 % by weight of at least one nonionic surfactant,
each calculated to the total composition.

3. Composition according to claim 1 or 2, comprising as water-soluble binding agent 15 % to 70 % by weight of maltodextrin.

4. Composition according to one or more of claims 1 to 3, comprising in addition about 1 % to 35 % by weight of urea.

5. Composition according to one or more of claims 1 to 4, comprising in addition 1 % to 35 % by weight of at least one alkaline earth salt.

6. Process for the preparation of a pulverulent composition according to one or more of claims 1 to 5, wherein an aqueous composition, comprising 5 to 30 parts by weight of an agent composition (i), present as aqueous emulsion, is mixed with an aqueous composition comprising 10 to 95 parts by weight of at least one water-soluble or water-dispersible binding agent, this mixture being homogenized at an increased temperature and subsequently spray-dried.

7. Use of a pulverulent composition according to one or more of claims 1 to 5 in hair care and hair treatment compositions.

## Revendications

1. Composition pulvérulente de substance active, qui contient:
(i) de 5 à 90 % d'une combinaison de
a) au moins un composé d'ammonium quaternaire de conditionnement des cheveux sélectionné parmi l'une des deux formules I ou II 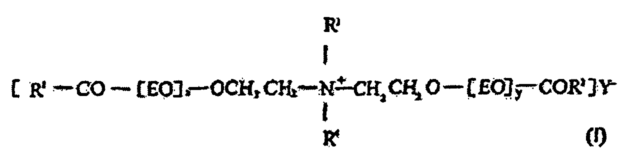 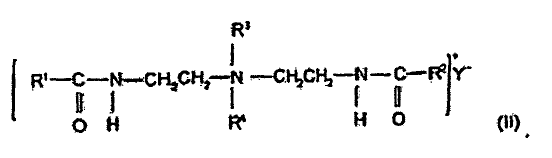 dans lesquelles R¹ et R² représentent chacun un groupe alkyle ou alcényle en C₈ à C₂₂, éventuellement hydroxylé, R³ et R⁴ un groupe alkyle en C₁ à C₃ ou un groupe -CH₂-CH₂-O-[EO]ₓ-H, x et y étant compris entre 0 et 5 et Y⁻ représente un anion, et
b) au moins un alcool gras en C₈ à C₂₄ et/ou une substance grasse ou huileuse, et
(ii) de 10 à 95 % en poids d'au moins un liant soluble dans l'eau ou dispersible dans l'eau, au moins 90 % des particules présentant un diamètre moyen compris entre environ 10 et 500 µm.

2. Composition selon la revendication 1, qui contient une combinaison (i) constituée de
a) de 1 à 35 % en poids d'au moins un composé d'ammonium quaternaire,
b) de 1 à 35 % en poids d'au moins un alcool gras en C₈ à C₂₄ et/ou d'une graisse ou d'une huile et
c) de 1 à 30 % en poids d'au moins un agent tensioactif non ionique,
ces pourcentages étant tous calculés par rapport à la composition de la combinaison.

3. Composition selon les revendications 1 ou 2, **caractérisée en ce que** comme liant soluble dans l'eau, elle contient de 15 à 70 % en poids de maltodextrine.

4. Composition selon l'une ou plusieurs des revendications 1 à 3, **caractérisée en ce qu'**elle contient en supplément environ 1 à 35 % en poids d'urée.

5. Composition selon l'une ou plusieurs des revendications 1 à 4, **caractérisée en ce qu'**elle contient en supplément de 1 à 35 % en poids d'au moins un sel de métal alcalino-terreux.

6. Procédé pour la préparation d'une composition pulvérulente selon l'une ou plusieurs des revendications 1 à 5, **caractérisée en ce que** l'on mélange une composition aqueuse qui contient de 5 à 30 parties en poids d'une combinaison (i) de substances actives sous forme d'émulsion aqueuse selon les revendications 1 à 5 avec une composition aqueuse qui contient de 10 à 95 parties en poids d'au moins un liant soluble dans l'eau ou dispersible dans l'eau, ce mélange étant homogénéisé à température plus élevée et ensuite séché par pulvérisation.

7. Utilisation d'une composition pulvérulente selon l'une ou plusieurs des revendications 1 à 5 dans des agents de soin et de traitement des cheveux.
